# EUROPEAN PATENT SPECIFICATION

(11) **EP 1 637 165 B1**
(45) Date of publication and mention of the grant of the patent: **24.12.2008**
(21) Application number: 05255818.6
(22) Date of filing: 20.09.2005
(51) Int. Cl.: A61K 47/38, A61K 9/107

(54) **Medicinal cooling emulsions**
Medizinische Emulsionen mit Kühleffekt
Emulsions médicinales à effet refroidissant

(30) Priority: 21.09.2004 US 945785
(43) Date of publication of application: 22.03.2006
(73) Proprietor: McNeil-PPC, Inc., Skillman, NJ 08558 (US)
(72) Inventor: Szymczak, Christopher E., Marlton, NJ 08053 (US)
(74) Representative: Mercer, Christopher Paul

(56) References cited:
- WO-A-97/42945
- WO-A-03/007908
- CA-A1- 2 461 275
- US-A- 4 044 120
- US-A- 4 190 643
- US-A- 5 458 879
- US-A- 5 498 426
- US-A- 5 560 913
- US-A1- 2002 119 110

## Description

### FIELD OF THE INVENTION

This invention relates to a novel emulsion containing a non volatile cooling agent. This invention further relates to liquid dosage forms containing a non-volatile cooling agent, a cellulosic polymer, and an aqueous vehicle, with at least one active ingredient dispersed therein.

### BACKGROUND OF THE INVENTION

A major concern in designing pharmaceutical dosage forms is making convenient, uniformly dispersed, palatable medications that facilitate patient compliance with the recommended dosing regimen. One of the most popular pharmaceutical dosage forms includes tablets that may be swallowed. It is common practice to coat such dosage forms with substances, such as film-forming polymers, fats, sugars, or gelatin, in order to facilitate swallowing ease, to hide an objectionable taste of the tablet, and/or to provide a perceptible pleasing taste to the tablet.

There are many disadvantages with solid dosage forms over liquid dosage forms. Children, elderly, and many other persons including disabled or incapacitated patients often experience difficulties in swallowing tablets. In these situations, it is desirable to provide the drug in liquid form because of the ease with which it may be swallowed. In addition, patients may be more inclined to comply with their medication instruction if the dosages are easily ingestible. Also, there is greater dosing flexibility with liquid preparations than with solid dosage forms.

Disadvantageously, liquid dosage forms often have stability problems associated with maintaining the drugs in suspension. If liquid pharmaceutical suspensions are poorly formulated, the drug settles out as a sediment, which thereby reduces the therapeutic concentration of drug in the suspension. As a result, the patient may be underdosed or overdosed, and the patient's recovery may be seriously compromised.

In addition to improving the ease with which a medication may be swallowed, another method for improving a patient's compliance with medication instructions is via designing a dosage form with superior taste, mouth feel, or other organoleptic characteristics, such as one that provides a sensory "cue" to the consumer that the medicine may be starting to work, are all known methods of obtaining a consumer-preferred product. Recently in the confectionary marketplace, mints, gums, and breath-freshening strips, which provide a cooling sensation in the mouth or throat, have also become especially popular with consumers.

In the pharmaceutical marketplace, cooling agents have also been used in dosage forms not only to satisfy the consumer's preference for a pleasant tasting form, but also to enhance the physiological and/or perceived benefits, e.g., speed of relief, duration of relief, and improved aesthetics of the medicine. For example, it is known to include volatile mint-like compounds, such as menthol or peppermint oil, in coatings for swallowable pharmaceutical tablets in order to provide the user with a cooling sensation. See, e.g., U.S. Patent No. 5,098,715 and U.S. Patent No. 5,827,852. Likewise, menthol, peppermint oil and other volatile cooling agents have been used commonly in liquid medicinal preparations for flavoring or taste-masking. These volatile coolants or cooling agents have also been employed with sweeteners in liquid cough-treatment compositions. See PCT Publication No. WO 02/45714. However, some high-intensity sweeteners, such as aspartame, are subject to degradation when heated.

Volatile compounds are often identifiable through detection of odor or quantitatively through weight loss under specified atmospheric conditions. This volatilization or odor signifies loss of flavor to the atmosphere thus rendering the product physically unstable from a flavor standpoint. Another limitation associated with the use of volatile mint-like compounds is the dietary restrictions regarding mint usage in certain patient populations, e.g. those with gastroesophageal reflux disease ("GERD"). Yet another limitation regarding the use of such volatile compounds is a perceived social stigma associated with the smell of mentholated medicine in public. Furthermore, dosage forms having a "minty" or menthol-like smell or odor may be confused with candies and mints or cough-drops. In the case of pets that rely on the sense of smell, or visually handicapped, this could also cause accidental ingestion of a medication or confusion with other items normally ingested.

Flavoring compounds also typically need to be dispersed through aqueous media through the use of a surfactant or surface agent. Often the addition of these agents (ie.,e.g., sodium lauryl sulfate, or polysorbate 80) alter the taste profile to a "soapy" or bitter. Sometimes a small amount of an alcohol-based cosolvent is also required. Disadvantageously, the use of such co-solvents also further impacts the taste of the cooling agents.

One method for overcoming the disadvantages associated with using volatile mint-like compounds in pharmaceutical dosage forms was disclosed in United States Serial No. 10/391,396 which disclosed a composition suitable for coating solid dosage forms containing a coating agent such as hydroxypropylmethylcellulose, a high intensity sweetener such as sucralose, and a menthyl ester non-volatile cooling agent.

Cooling agents have been also been employed into chewable dosage form in order to create a prolonged cooling sensation in the throat. See PCT Publication No. 97/24036. However, such chewable dosage forms are designed to remain in the mouth for some period of time and may not disintegrate or dissolve completely upon chewing. Not only may this retard dissolution of the active ingredient, but it also may delay onset of the active.

A need therefore remains for an economic, viscous, stable, liquid dosage form that provides a pleasant cooling sensation substantially absent of any odor. A need further remains for such dosage forms that are substantially free of volatile compounds and do not require the inclusion of a surfactant or alcohol-based cosolvent.

US 4,044,120, US 4,190,643, US 2002/119110 and WO 03/007908 all describe liquid formulations comprising a cellulosic polymer and a non-volatile cooling agent.

### SUMMARY OF THE INVENTION

The present invention describes an emulsion comprised of, consisting of, and/or consisting essentially of a menthyl ester non-volatlle cooling agent, a cellulosic polymer emulsifier, and water, as well as a liquid dosage form containing the same as defined in the claims.

The present invention provides a novel emulsion system for use in orally-administered liquid pharmaceutical dosage forms, which are stable and pourable. The resulting liquid pharmaceutical dosage form further provides the user with a mild, pleasant, long-lasting cooling sensation in the mouth and throat during ingestion without any substantial aroma or olfactory stimulation and without the negative taste effects associated with the use of surfactants or alcohol-based co-solvents.

Other features and advantages of the present invention will be apparent from the detailed description of the invention and from the claims.

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. As used herein, all percentages are by weight unless otherwise specified.

As used herein, the term "dosage form" applies to any composition designed to contain a specific pre-determined amount or "dose" of a certain ingredient, for example an active ingredient as defined below. Dosage forms of the present invention are typically liquid, and may include, but are not limited to: a) pharmaceutical drug delivery systems, including those for oral administration, nasal administration, or buccal administration; or b) compositions for delivering minerals, vitamins and other nutraceuticals, oral care agents. Furthermore, the dosage forms of the present invention may also include swallowable liquid-filled dosage forms which have a liquid core. In one embodiment, the dosage form is an orally administered system for delivering a pharmaceutical active ingredient to the GI tract. In another embodiment, the dosage form is a nasally administered system for delivering a pharmaceutical active ingredient to the nasal mucosa or sinuses. In another embodiment, the dosage form is topically administered to the oral or pharyngeal mucosa in the form of a spray or swabbed liquid.

"Emulsifying agent" or "emulsifier," as used herein refers to a substance that forms an emulsion when added to two immiscible liquids.

"Water soluble" as used herein in connection with non-polymeric materials, shall mean from sparingly soluble to very soluble, i.e., not more than 100 parts water required to dissolve 1 part of the non-polymeric, water soluble solute. See Remington, "The Science and Practice of Pharmacy," pages 208 - 209 (2000). "Water soluble" as used herein in connection with polymeric materials, shall mean that the polymer swells in water and can be dispersed at the molecular level to form a homogeneous dispersion.

"Cooling agents," as used herein, include solid or liquid substances that inhibit heat receptors or stimulate cooling receptors located on the free-nerve endings of the CN V trigeminal nerve. In one embodiment, the cooling agents provide a sensory cooling effect, either immediate or delayed, to the user without significant interaction with one or more of the taste sensors such as bitter, sour, sweet, umami, or salty.

"Non-volatile cooling agents," as used herein, shall represent a subgroup of cooling agents comprised of one or more individual chemical compounds that are substantially free from odor and odorless vapor such that they a) do not lose more than about 1% by weight when placed in an open container at 50°C for at least one hour; and usually b) have an average molecular weight of greater than 300 atomic mass (or molecular) units (amu) or more as described by the "The Royal Society of Chemistry" website, London UK (www.chemsoc.org/exemplarchem/entrles/2001/caphane/flavour.html , 2002). "Average molecular weight," as used herein, shall mean a mathematical weighted average of all of the individual components weighted according to the weight fraction or percent concentration in solution as defined in Martin, Physical Pharmacy, 561 (4th Ed. 1993)(also referred to as "weight-average molecular weight").

"Emulsion" as used herein refers to a thermodynamically unstable but physically stable liquid composition containing an oil soluble liquid phase and a water soluble liquid phase, wherein one phase is intimately and uniformly dispersed throughout the other phase in the form of small droplets or globules. The emulsion typically has a continuous phase (or external phase) and a dispersed phase (or internal phase). As used herein, a "continuous" phase is a substantially homogenous bulk liquid phase, which is either primarily polar (hydrophilic) or nonpolar (hydrophobic) in nature. A "dispersed" phase is a substantially homogenous liquid phase that forms a distinct layer with the continuous/external phase in the absence of an emulsifying agent. One type of emulsion is an "oil-in-water (o/w) emulsion," which is an emulsion where the continuous phase is primarily polar and the dispersed phase is nonpolar. Another type of emulsion is a "water-in-oil (w/o) emulsion," which is an emulsion where the continuous phase is primarily nonpolar and the dispersed phase is polar. See Martin, A., Physical Pharmacy, 486-496 (4th ed. 1993).

The first embodiment of this invention is directed to an emulsion composition including, based upon the total weight of the emulsion composition, a) from about 0.001 percent to about 20 percent, e.g. from about 1 percent to about 20 percent of a cellulosic polymer emulsifier; b) from about 0.0001 percent to about 40 percent, e.g. from about 0.01 percent to about 15 percent of a non-volatile cooling agent; and c) from about 50 percent to about 99 percent of water.

In one embodiment, the emulsion composition is substantially free of volatile cooling agents such as mint and menthol. "Substantially free of volatile cooling agents," as used herein, shall mean inclusion of less 0.1 percent, e.g., less than 0.01 percent, of volatile cooling agents as based upon the total weight of the emulsion composition.

Suitable cellulosic polymers include but are not limited to, methylcellulose, hydroxypropylcellulose (HPC), hydroxyethylmethylcellulose (HEMC), hydroxypropylmethylcellulose (HPMC), hydroxybutylmethylcellulose (HBMC), cellulose acetate (CA), cellulose acetate phthalate (CAP), carboxymethylcellulose (CMC), hydroxyethylcellulose (HEC), hydroxythylethylcellulose (HEEC), hydroxyethylhydroxypropylmethyl cellulose (HEMPMC), and polymers, and derivatives and mixtures thereof.

One suitable hydroxypropylmethylcellulose compound is "HPMC 2910", which is a cellulose ether having a degree of substitution of about 1.9 and a hydroxypropyl molar substitution of 0.23, and containing, based upon the total weight of the compound, from about 29% to about 30% methoxyl and from about 7% to about 12% hydroxypropyl groups. HPMC 2910 is commercially available from the Dow Chemical Company under the tradename, "Methocel E" or "Methocel E5, " which is one grade of HPMC-2910 suitable for use in the present invention, has a viscosity of about 4 to 6 cps (4 to 6 millipascal-seconds) at 20 °C in a 2% aqueous solution as determined by a Ubbelohde viscometer, Similarly, "Methocel E6 ," which is another grade of HPMC-2910 suitable for use in the present invention, has a viscosity of about 5 to 7 cps (5 to 7 millipascal-seconds) at 20 °C in a 2% aqueous solution as determined by a Ubbelohde viscometer. "Methocel E15," which is another grade of HPMC-2910 suitable for use in the present invention, has a viscosity of about 15000 cps (15 millipascal-seconds) at 20 °C in a 2% aqueous solution as determined by a Ubbelohde viscometer. As used herein, "degree of substitution" shall mean the average number of substituent groups attached to an anhydroglucose ring, and "hydroxypropyl molar substitution" shall mean the number of moles of hydroxypropyl per mole anhydroglucose.

Another suitable microcrystalline cellulose is a dried coprecipitated microcrystal of cellulose and carboxymethyl cellulose, Sodium carboxymethyl cellulose is commonly used as the coprecipitate in microcrystalline cellulose. The microcrystalline cellulose may contain, based upon the total weight of the microcrystalline cellulose, from about 8 percent to about 19 percent, or about 8 percent to about 14 percent, of carboxymethyl cellulose, such as sodium carboxymethyl cellulose. Microcrystalline cellulose as described above is commercially available from FMC under the trademark, "Avicel™".

Suitable non-volatile cooling agents are menthyl esters. One example of a suitable non-volatile cooling agents is the menthyl ester mixture commercially available from International Flavors & Fragrances under the tradename, "Cooler #2".

In one embodiment, the emulsion of the present invention may contain the cellulosic polymer and non-volatile cooling agent in a weight ratio of about 25:1 to about 1:25, e.g., from about 10:1 to about 1:10.

Optionally, the emulsion composition may contain, based upon the total weight of the emulsion composition, from greater than about 0 percent to less than about 49 percent of an alcohol such as, for example, ethanol, glycerol, polyols such as propylene glycol, and mixtures thereof.

In one embodiment, the composition of the present invention may further contain one or more active ingredients. The term "active ingredient" is used herein in a broad sense and may encompass any material that can be carried by or entrained in the system. For example, the active ingredient can be a pharmaceutical, nutraceutical, vitamin, dietary supplement, nutrient, herb, dyestuff, nutritional, mineral, supplement, or the like and combinations thereof.

The dosage forms of the present invention contain a safe and effective amount of the active ingredient, which means an amount of the agent that Is high enough, when administered, to significantly positively modify the condition to be treated or prevent an adverse or unwanted condition through short-term Immediate use or repeated long-term chronic use used within the scope of sound medical judgment. The safe and effective amount of the active ingredient will vary with the particular condition being treated; the physical condition and age of the patient being treated; the nature of concurrent therapy, if any; the duration of the treatment; the particular carrier utilized; the method of administration; the specific active ingredient(s) employed; and the like. Typically, the active ingredient(s) are used in an amount, based upon the total weight of the dosage form, from about 0.001 percent to about 99.9 percent, e.g. from about 0.1 percent to about 75 percent.

The active ingredient or ingredients may be present in the dosage form in a variety of forms. For example, the active ingredient(s) may be in the form of particles, which in turn may be coated or uncoated. Suitable coatings for the particles include any of those set forth in the art such as, for example, those set forth in Lachman, Lieberman, and Kanig, The Theory and Practice of Industrial Pharmacy, 3rd Ed. Section 3, 359-372 (1986). If the active ingredient is in form of particles, the particles (whether coated or uncoated) typically have an average particle size of about 1 micron to about 2000 microns. The active ingredient may also be in the form of a solid suspended in the emulsion of the present invention, or may be substantially dissolved in the continuous phase.

Suitable pharmaceuticals include analgesics, anti-inflammatory agents, antiarthritics, anesthetics, antihistamines, antitussives, antibiotics, anti-infective agents, antivirals, anticoagulants, antidepressants, antidiabetic agents, antiemetics, antiflatulents, antifungals, antispasmodics, appetite suppressants, bronchodilators, cardiovascular agents, central nervous system agents, central nervous system stimulants, decongestants, oral contraceptives, diuretics, expectorants, gastrointestinal agents, migraine preparations, motion sickness products, mucolytics, muscle relaxants, osteoporosis preparations, polydimethylsiloxanes, respiratory agents, sleep-aids, urinary tract agents, oral care agents,flavorants, and mixtures thereof.

Suitable oral care agents include breath fresheners, tooth whiteners, antimicrobial agents, tooth mineralizers, tooth decay inhibitors, topical anesthetics, mucoprotectants, and the like.

Suitable flavorants include menthol, peppermint, mint flavors, fruit flavors, chocolate, vanilla, bubblegum flavors, coffee flavors, liqueur flavors and combinations and the like.

Examples of suitable gastrointestinal agents include antacids such as calcium carbonate, magnesium hydroxide, magnesium oxide, magnesium carbonate, aluminum hydroxide, sodium bicarbonate, dihydroxyaluminum sodium carbonate; stimulant laxatives, such as bisacodyl, cascara sagrada, danthron, senna, phenolphthalein, aloe, castor oil, ricinoleic acid, and dehydrocholic acid, and mixtures thereof; H2 receptor antagonists, such as famotadine, ranitidine, cimetadine, nizatidine; proton pump inhibitors such as omeprazole or lansoprazole; gastrointestinal cytoprotectives, such as sucraflate and misoprostol; gastrointestinal prokinetics, such as prucalopride, antibiotics for H. pylori, such as clarithromycin, amoxicillin, tetracycline, and metronidazole; antidiarrheals, such as diphenoxylate and loperamide; glycopyrrolate; antiemetics, such as ondansetron, analgesics, such as mesalamine; and antiflatulants, such as polydimethylsiloxanes. Examples of suitable polydimethylsiloxanes, which include, but are not limited to dimethicone and simethicone, are those disclosed in United States Patent Nos. 4,906,478, 5,275,822, and 6,103,260. As used herein, the term "simethicone" refers to the broader class of polydimethylsiloxanes, including but not limited to simethicone and dimethicone.

In one embodiment of the invention, the active ingredient may be selected from bisacodyl, famotadine, ranitidine, cimetidine, prucalopride, diphenoxylate, loperamide, lactase, mesalamine, bismuth, antacids, and pharmaceutically acceptable salts, esters, isomers, and mixtures thereof.

In another embodiment, the active ingredient may be a gastrointestinal agent selected from laxatives, H2 receptor antagonists, proton pump inhibitors, gastrointestinal cytoprotectives, gastrointestinal prokinetics, anitbiotics, antidiarrheals, and antiemetics.

In another embodiment, the active ingredient is selected from analgesics, anti-inflammatories, and antipyretics, e.g. non-steroidal anti-inflammatory drugs (NSAIDs), including propionic acid derivatives, e.g. ibuprofen, naproxen, ketoprofen and the like; acetic acid derivatives, e.g. indomethacin, diclofenac, sulindac, tolmetin, and the like; fenamic acid derivatives, e.g. mefenamic acid, meclofenamic acid, flufenamic acid, and the like; biphenylcarbodylic acid derivatives, e.g. diflunisal, flufenisal, and the like; and oxicams, e.g. piroxicam, sudoxicam, isoxicam, meloxicam, and the like. In one particular embodiment, the active ingredient is selected from propionic acid derivative NSAID, e.g. ibuprofen, naproxen, flurbiprofen, fenbufen, fenoprofen, indoprofen, ketoprofen, fluprofen, pirprofen, carprofen, oxaprozin, pranoprofen, suprofen, and pharmaceutically acceptable salts, derivatives, and combinations thereof. In another particular embodiment of the invention, the active ingredient may be selected from acetaminophen, acetyl salicylic acid, ibuprofen, naproxen, ketoprofen, flurbiprofen, diclofenac, cyclobenzaprine, meloxicam, rofecoxib, celecoxib, and pharmaceutically acceptable salts, esters, isomers, and mixtures thereof.

In another embodiment of the invention, the active ingredient may be selected from pseudoephedrine, phenylpropanolamine, chlorpheniramine, dextromethorphan, diphenhydramine, astemizole, terfenadine, fexofenadine, loratadine, desloratadine, cetirizine, mixtures thereof and pharmaceutically acceptable salts, esters, isomers, and mixtures thereof.

The dosage form may also further optionally comprise other ingredients such as, based upon the total weight of the dosage form, from about 00 percent to about 70 percent of sweeteners, from about 0 percent to about 1 percent of preservatives such as parabens; from about 0 percent to about 5 percent of opacifying agents such as titanium dioxide; and/or from about 0 percent to about 15 percent colorants. See Remington's Practice of Pharmacy, Martin & Cook, 17th ed., pp. 1625 - 30.

Examples of suitable sweeteners include those disclosed in United States Patent No. 5,272,137, and further may include heat-stable, high-intensity sweeteners. "Heat-stable, high-intensity sweeteners," as used herein, shall include chemical compounds or mixtures of compounds which elicit a sweet taste at least five times sweeter than sucrose, as measured in accordance with the test method described in G.B. Patent No. 1,543,167. Typically such sweeteners are substantially free from degradants after being heated for about one hour at about 40 °C. Examples of such suitable sweeteners include, but are not limited to, sucralose, neotame, and mixtures thereof.

Sucralose, which is also known as 4,1,6'-trideoxy-galactosucrose, is a heat-stable, high-intensity sweetener that may be produced in accordance with the process disclosed in U.K. Patent No. 1,544,167, and U.S. Patent Nos. 5,136,031 and 5,498,709.

Neotame which is also known as N-(N-(3,3-dimethylbutyl)-L-α-aspartyl)-L-phenylalanine 1 methyl ester, a derivative of the dipeptide composed of the amino acids, aspartic acid and phenylalanine, is a heat-stable, high-intensity sweetener which was approved for use in the United States, July 2002 and is commercially available from The NutraSweet® Company.

Coloring agents should be selected to avoid chemical incompatibilities the other ingredients in the dosage form. Suitable coloring agents for use in pharmaceutical applications may be used in the present invention and may include, but not be limited to azo dyes, quinopthalone dyes, triphenylmethane dyes, xanthene dyes, indigoid dyes, iron oxides, iron hydroxides, titanium dioxide, natural dyes, and mixtures thereof. More specifically, suitable colorants include, but are not limited to patent blue V, acid brilliant green BS, red 2G, azorubine, ponceau 4R, amaranth, D&C red 33, D&C red 22, D&C red 26, D&C red 28, D&C yellow 10, FD&C yellow 5, FD&C yellow 6, FD&C red 3, FD&C red 40, FD&C blue 1, FD&C blue 2, FD&C green 3, brilliant black BN, carbon black, iron oxide black, iron oxide red, iron oxide yellow, titanium dioxide, riboflavin, carotenes, antyhocyanines, turmeric, cochineal extract, clorophyllin, canthaxanthin, caramel, betanin, and mixtures thereof. Preservatives useful in the present invention include but are not limited to sodium benzoate, potassium sorbate, salts of edetate (also know as salts of ethylenediaminetetraacetic acid, or EDTA, such as disodium edetate) and parabens (such as methyl, ethyl, propyl and butyl p- hydroxybenzoic acids esters). The preservatives listed above are exemplary, but each preservative must be evaluated on an empirical basis, in each formulation, to assure the compatibility and efficacy of the preservative. Methods for evaluating the efficacy of preservatives in pharmaceutical formulations are known to those skilled in the art.

Preservatives are generally present in amounts of up to gram per 100 mL of the emulsion, or from about 0.15 to about 0.5 grams per 100 mL of the emulsion. For example, in pharmaceutical emulsions containing acetaminophen, sodium benzoate may be present in the range of from about 0.15 to about 0.3 grams, or from about 0.20 grams to about 0.3 grams per 100 mL of the emulsion, and butylparaben may be present in the range of from about 0.01 to about 0.05 grams, or from about 0.025 grams to about 0.05 grams per 100 mL of the emulsion.

In one embodiment, at least about 50 percent of the non-volatile cooling agent and/or at least about 90 percent of the cellulosic polymer is contained within the dispersed phase of the emulsion of the present invention.

In one embodiment, the liquid composition of the present invention may be prepared by first combining the cooling agent with the emulsifier and the water under ambient conditions until the resulting mixture is a visually homogeneous emulsion. Then, the desired pharmaceutical agent, as well as any other optional ingredients, may be added thereto with mixing under ambient conditions.

Alternatively, in order to improve the uniform distribution of the emulsifier, the water with optional alcohol may be heated to a temperature of about 70°C to about 85 °C, then the emulsifier may be added thereto with stirring. After the resulting mixture is homogeneous, the cooling agent may be added thereto, either with or without heating. The pharmaceutical active ingredient, as well as any other optional ingredients, may then be added thereto with mixing under ambient conditions.

The pharmaceutical dosage forms of the present invention may be used to treat the symptoms of headaches, sinusitis, cough, cold, flu allergy, and the like.

Applicants unexpectedly found that when an active ingredient is combined with the emulsion of the present invention, the resulting dosage form remained in a stable, pourable form, and upon oral administration, provided the user with uniform cooling characteristics. Applicants further unexpectedly found that at low shear rates, the viscosity of the emulsion of the present invention was significantly lower than the viscosity of the combination of equal amounts of cooling agent in water, or the combination of equal amounts of cellulosic polymer in water, respectively. In view of this lower viscosity value, when the liquid emulsion is poured, sprayed or squirted into the desired mucosal region, it spreads and coats the mucosal surface evenly.

Furthermore, it was unexpectedly found that as the shear rate applied to the liquid mixture increased in an amount comparable to that which might be applied to the product during shaking by the user, the viscosity of the resulting mixture did not substantially change. Surprisingly, the standard deviation of the viscosity of the resulting mixture also was lower than that reported for either the cooling agent alone or the cellulosic polymer alone when tested at the same concentrations as used in the combined mixture. This demonstrable synergy between the cooling agent and the cellulosic polymer suggested that the resulting liquid mixture is a unique, intimately mixed liquid having a viscosity that is less responsive to shearing (or any mechanically applied force) than the individual cooling agent and cellulosic polymers therein, respectively. This is particularly beneficial to the user, who can therefore expect to receive the same performance properties regardless of the amount of physical handling, e.g shaking by the patient or mechanical vibrations/bouncing through shipment previously applied to the product.

As a result of this unexpected viscosity profile, the dosage form was capable of evenly coating the throat upon oral ingestion and thus providing a long-lasting, mild cooling sensation in the throat and/or mouth without any associated, unpleasant aroma/odor or polarizing taste as may be experienced by use of coatings containing menthol and other intense mint-like volatile flavors. Surprisingly, the cooling sensation, which primarily occurred after swallowing, could also be "reactivated" or "re-intensified" through choice by the user for several minutes after consumption by simply taking a slightly deeper or slightly exaggerated breath despite the absence of the solid dosage form in the mouth or throat.

Similarly, the dosage form of the present invention, which may optionally contain saline, is capable of evenly coating the nasal mucosa upon nasal administration and thus providing a long-lasting, mild cooling sensation in the nasal region without any associated, unpleasant aroma/odor.

Yet a further advantage of the present invention is that because the emulsion was substantially free of volatile cooling agents such as mints, it provided the user with a cooling benefit when swallowed or ingested without aggravating conditions such as gastroesophageal reflux disease commonly referred to as "GERD".

The invention illustratively disclosed herein suitably may be practiced in the absence of any component, ingredient, or step which is not specifically disclosed herein. Several examples are set forth below to further illustrate the nature of the invention and the manner of carrying it out. However, the invention should not be considered as being limited to the details thereof.

### EXAMPLES

### Example 1: Comparison of Viscosities of CellulosicPolymer, Cooling Agent, Water, and Combinations thereof

The compositions having the formulas set forth below in Table A were prepared as follows:

### Preparation of Formula A

After heating the water to a temperature of about 70°C in a beaker, the cooling agent was added thereto with stirring. After the mixture was visually homogeneous, the mixture was cooled to room temperature.

### Preparation of Formula B

After heating the water to a temperature of about 70°C in a beaker, the cellulosic polymer was added thereto with stirring. After the mixture was visually homogeneous, the mixture was cooled to room temperature.

### Preparation of Formula D

After heating the water to a temperature of about 70°C in a beaker, the cellulosic polymer was added thereto with stirring. After the mixture was visually homogeneous, cooling agent was added thereto with stirring. After the mixture was visually homogeneous, the mixture was cooled to room temperature.

**Table A: Compositions for Viscosity Testing**

| Ingredient | Formula A | Formula B | Formula C | Formula D |
|---|---|---|---|---|
| Cooler #2, SN069450 IFF Inc. | 5 | 0 | 100 | 5 |
| Hypromellose E-5, Methocel™ Dow Chemical Co. | 0 | 8 | 0 | 8 |
| Purified Water, USP | 95 | 92 | 0 | 87 |

| | | | | |
|---|---|---|---|---|
| All percentages expressed as w/w%. | | | | |

### Measurement of Viscosity

A 10 ml sample of Formula A was placed into a_Brookfield Digital Viscometer Model DV-II, #31 spindle, having a temperature of cup of 25°C at 0.3 RPM. This procedure was independently repeated for the same formula, but at various RPM (0.6, 1.5, 3, 6, 12, 30).

This procedure was also independently repeated for samples of Formulas B through D, respectively. The results are set forth below in Table B, in which all viscosity values are set forth in centipoise (cps):

**Table B: Comparison of Viscosities**

| RPM | Formula A* | Formula B | Formula C | **Formula D**** |
|---|---|---|---|---|
| 0.3 | 1000 | 802 | 902 | **401** |
| 0.6 | 351 | 451 | 601 | **200** |
| 1.5 | 40.1 | 210 | 461 | **110** |
| 3 | 0 | 140 | 471 | **100** |
| 6 | 0 | 105 | 496 | **105** |
| 12 | 0 | 95.2 | 498 | **108** |
| 30 | 0 | 90.2 | 508 | **110** |
| **AVERAGE (cps)** | **464** | **271** | **562** | **162** |
| **Maximum Viscosity (cps)** | **1000** | **802** | **902** | **401** |
| **Minimum Viscosity (cps)** | **40.1** | **90.2** | **461** | **100** |
| **Viscosity Range (cps)***** | **959.9** | **711.8** | **441** | **301** |
| **Standard Deviation (cps)** | **376** | **267** | **157** | **111** |

| | | | | |
|---|---|---|---|---|
| *Note: viscosity measurement of "0" not included as part of average, minimum viscosity or standard deviation.* *Cooler #2 ingredient observed separate from water phase, **White, opaque physically stable emulsion. ***Calculated difference between the maximum and minimum values . | | | | |

This Example showed that at lower shear rates (0.3 rpm to 3 rpm), Formula D demonstrated a lower viscosity (or was "thinner") than either the cooling agent in water (Formula A), the cellulosic polymer in water (Formula B), or the cooling agent alone. As a result of having this low viscosity property, the emulsion of the present invention may be particularly effective in, for example, evenly coating and spreading across the throat upon ingestion.

This Example also showed that Formula D maintained a viscosity between 105 and 110 cps for shear rates 1.5 rpm to 30 rpm. This consistency in viscosity is particularly advantageous to providing the user with the same product performance expectations regardless of how the product was physically handled.

## Claims

1. An emulsion for use in orally-administered liquid pharmaceutical dosage forms comprising, based upon the total weight of the emulsion:
a. from 0.001 percent to 20 percent of a cellulosic polymer;
b. from 0.0001 percent to 40 percent of a non-volatile cooling agent having a water solubility of less than about 0.001% by weight; and
c. from 50 to 99 percent water,
wherein the weight ratio of cellulosic polymer to non-volatile cooling agent is from 25:1 to 1:25, wherein the emulsion has a viscosity of about 100 cps when measured In a Brookfield viscometer (Spindle #31) at a temperature of 25 °C and a speed of 1.5 rpm to 30 rpm, and wherein the non-volatile cooling agent is a menthyl ester.

2. A pharmaceutical dosage form comprising the emulsion of Claim 1 and further comprising a pharmaceutically active ingredient.

3. The pharmaceutical dosage form of Claim 2, wherein the active ingredient is selected from analgesics, antihistamines, decongestants, cough suppressants, expectorants, gastrointestinal agents, chemotherapeutic agents, antibiotics, and combinations thereof.

4. The emulsion of any of Claims 1 to 3, which is substantially free of volatile cooling agents.

5. The emulsion of any of Claims 1 to 4, having a continuous phase and a dispersed phase, wherein the continuous phase is aqueous and wherein at least about 50 percent of the non-volatile cooling agent is contained within the dispersed phase.

6. The emulsion of any of Claims 1 to 5, wherein the cellulosic polymer is selected from methylcellulose, hydroxypropylcellulose, hydroxyethylmethylcellulose, hydroxypropylmethylcellulose, carboxymethylcellulose, microcrystalline cellulose, and copolymers, and derivatives and mixtures thereof.

7. The emulsion of any of claims 1 to 6, wherein the weight ratio of cellulosic polymer to non-volatile cooling agent is from 1:10 to 10:1.

8. The pharmaceutical dosage form of claim 2 or 3 for use in treating the symptoms of headache, sinusitis, cough, cold, allergy, and/or flu in a mammal comprising orally administering to the mammal in need of such treatment.

9. The pharmaceutical dosage form of claim 2 or 3 for use in treating the symptoms of headache, sinusitis, cough, cold, allergy, and/or flu in a mammal comprising intranasally administering to the mammal in need of such treatment.

## Patentansprüche

1. Emulsion zur Verwendung in oral verabreichten flüssigen pharmazeutischen Dosierungsformen, umfassend, bezogen auf das Gesamtgewicht der Emulsion:
a. von 0,001 Prozent bis 20 Prozent eines Cellulosepolymers;
b. von 0,0001 Prozent bis 40 Prozent eines nicht-flüchtigen Kühlmittels mit einer Wasserlöslichkeit von weniger als etwa 0,001 Gew.-%; und
c. von 50 bis 99 Prozent Wasser,
wobei das Gewichtsverhältnis von Cellulosepolymer zu nicht-flüchtigem Kühlmittel von 25:1 bis 1:25 beträgt, wobei die Emulsion eine Viskosität von etwa 100 cps besitzt, wenn gemessen in einem Brookfield-Viskometer (Spindel #31) bei einer Temperatur von 25°C und einer Geschwindigkeit von 1,5 UPM bis 30 UPM, und wobei das nicht-flüchtige Kühlmittel ein Menthylester ist.

2. Pharmazeutische Dosierungsform, die die Emulsion nach Anspruch 1 umfasst und weiter einen pharmazeutisch wirksamen Inhaltsstoff umfasst.

3. Pharmazeutische Dosierungsform nach Anspruch 2, wobei der wirksame Inhaltsstoff ausgewählt ist aus Analgetika, Antihistaminika, Entstauungsmitteln, Hustenunterdrückungsmitteln, Expektorantien, gastrointestinalen Mitteln, chemotherapeutischen Mitteln, Antibiotika und Kombinationen davon.

4. Emulsion nach einem der Ansprüche 1 bis 3, die im wesentlichen frei von flüchtigen Kühlmitteln ist.

5. Emulsion nach einem der Ansprüche 1 bis 4, mit einer kontinuierlichen Phase und einer dispergierten Phase, wobei die kontinuierliche Phase wässrig ist und wobei wenigstens etwa 50 Prozent des nicht-flüchtigen Kühlmittels in der dispergierten Phase enthalten sind.

6. Emulsion nach einem der Ansprüche 1 bis 5, wobei das Cellulosepolymer ausgewählt ist aus Methylcellulose, Hydroxypropylcellulose, Hydroxyethylmethylcellulose, Hydroxypropylmethylcellulose, Carboxymethylcellulose, mikrokristalliner Cellulose und Copolymeren und Derivaten und Mischungen davon.

7. Emulsion nach einem der Ansprüche 1 bis 6, wobei das Gewichtsverhältnis von Cellulosepolymer zu nicht-flüchtigem Kühlmittel von 1:10 bis 10:1 beträgt.

8. Pharmazeutische Dosierungsform nach Anspruch 2 oder 3 zur Verwendung bei der Behandlung der Symptome von Kopfschmerz, Sinusitis, Husten, Erkältung, Allergie und/oder Grippe in einem Säuger, welche die orale Verabreichung an den Säuger, der einer solchen Behandlung bedarf, umfasst.

9. Pharmazeutische Dosierungsform nach Anspruch 2 oder 3 zur Verwendung bei der Behandlung der Symptome von Kopfschmerz, Sinusitis, Husten, Erkältung, Allergie und/oder Grippe in einem Säuger, welche die intranasale Verabreichung an den Säuger, der einer solchen Behandlung bedarf, umfasst.

## Revendications

1. Émulsion destinée à être utilisée dans des formes posologiques pharmaceutiques liquides administrées par voie orale, qui comprend, par rapport au poids total de l'émulsion :
a.de 0,001 % à 20 % d'un polymère cellulosique ;
b. de 0,0001 % à 40 % d'un agent de refroidissement non volatil ayant une solubilité dans l'eau inférieure à environ 0,001 % en poids, et
c. de 50 à 99 % d'eau,
dans laquelle le rapport en poids du polymère cellulosique sur l'agent de refroidissement non volatil est de 25/1 à 1/25, dans laquelle l'émulsion a une viscosité d'environ 100 cps lorsqu'elle est mesurée dans un viscosimètre Brookfield (cylindre #31) à une température de 25°C et à une vitesse de 1,5 tr/minute à 30 tr/minute, et dans laquelle l'agent de refroidissement non volatil est un ester de menthyle.

2. Forme posologique pharmaceutique comprenant l'émulsion selon la revendication 1, et comprenant en outre un ingrédient pharmaceutiquement actif.

3. Forme posologique pharmaceutique selon la revendication 2, dans laquelle l'ingrédient est choisi parmi les analgésiques, les antihistaminiques, les décongestionnants, les antitussifs, les expectorants, les agents gastro-intestinaux, les agents chimiothérapeutiques, les antibiotiques, et les combinaisons de ceux-ci.

4. Émulsion selon l'une quelconque des revendications 1 à 3, qui est substantiellement dépourvue d'agents de refroidissement volatils.

5. Émulsion selon l'une quelconque des revendications 1 à 4, ayant une phase continue et une phase dispersée, dans laquelle la phase continue est aqueuse et dans laquelle au moins environ 50 % de l'agent de refroidissement non volatil est contenu à l'intérieur de la phase dispersée.

6. Émulsion selon l'une quelconque des revendications 1 à 5, dans laquelle le polymère cellulosique est choisi parmi la méthylcellulose, l'hydroxypropylcellulose, l'hydroxyéthylméthylcellulose, l'hydroxypropylméthylcellulose, la carboxyméthylcellulose, la cellulose microcristalline, et les copolymères, et les dérivés et mélanges de ceux-ci.

7. Émulsion selon l'une quelconque des revendications 1 à 6, dans laquelle le rapport en poids du polymère cellulosique sur l'agent de refroidissement non volatil est de 1/10 à 10/1.

8. Forme posologique pharmaceutique selon la revendication 2 ou 3, destinée à être utilisée dans le traitement de symptômes des céphalées, de la sinusite, de la toux, d'un rhume, d'une allergie, et/ou de la grippe chez un mammifère comprenant l'administration par voie orale au mammifère nécessitant un tel traitement.

9. Forme posologique pharmaceutique selon la revendication 2 ou 3, destinée à être utilisée dans le traitement de symptômes des céphalées, de la sinusite, de la toux, d'un rhume, d'une allergie, et/ou de la grippe chez un mammifère comprenant l'administration par voie intranasale au mammifère nécessitant un tel traitement.
